# EUROPEAN PATENT APPLICATION

(11) **EP 2 314 291 A1**
(43) Date of publication of application: **27.04.2011**
(21) Application number: 09305999.6
(22) Date of filing: 22.10.2009
(51) Int. Cl.: A61K 31/137, A61K 31/16, A61P 25/00

(54) **Use of the combination of teriflunomide and glatiramer acetate for treating multiple sclerosis**

(71) Applicant: Sanofi-Aventis, 75013 Paris (FR)
(72) Inventor: Byrnes, William, Bridgewater NJ 08807 (US); Douillet, Patrice, 75013, Paris (FR); Frangin, Gérald, 75013, PARIS (FR)
(74) Representative: Gaslonde, Aude

(57) **Abstract**

This invention is related to the use of the combination of teriflunomide or a pharmaceutically acceptable salt thereof and glatiramer acetate for treating multiple sclerosis.

## Description

### Field of the Invention

This invention is related to the use of the combination of teriflunomide or a pharmaceutically acceptable salt thereof and glatiramer acetate for treating multiple sclerosis.

### Background of the Invention

Multiple sclerosis (MS) is a debilitating, inflammatory, neurological illness characterized by demyelination of the central nervous system. The disease primarily affects young adults with a higher incidence in females. Symptoms of the disease include fatigue, numbness, tremor, tingling, dysesthesias, visual disturbances, dizziness, cognitive impairment, urologic dysfunction, decreased mobility, and depression. Four types classify the clinical patterns of the disease: relapsing-remitting, secondary progressive, primary-progressive and progressive-relapsing (S.L. Hauser and D.E. Goodkin, Multiple Sclerosis and Other Demyelinating Diseases in Harrison's Principles of Internal Medicine 14th Edition, vol. 2, Mc Graw-Hill, 1998, pp. 2409-2419).

The exact etiology of MS is unknown; however, it is strongly suspected that the demyelination characteristic of the disease is the result of an autoimmune response perhaps triggered by an environmental insult, e.g. a viral infection. Specifically, it is hypothesized that MS is caused by a T-cell-mediated, autoimmune inflammatory reaction. The autoimmune basis is strongly supported by the fact that antibodies specific to myelin basic protein (MBP) have been found in the serum and cerebrospinal fluid of MS patients and these antibodies along with T-cells that are reactive to MBP and other myelin proteolipids increase with disease activity. Furthermore, at the cellular level it is speculated that T-cell proliferation and other cellular events, such as activation of B cells and macrophages and secretion of cytokines accompanied by a breakdown of the blood-brain barrier can cause destruction of myelin and oligodendrocytes. (R.A. Adams, M.V. Victor and A.H. Ropper eds, Principles of Neurology, Mc Graw-Hill, New York, 1997, pp.903-921). Progressive MS (primary and secondary) may be based on a neurodegenerative process occurring with demyelination.

The use of (Z)-2-cyano-3-hydroxy-but-2-enoic acid-(4'-trifluoromethylphenyl)-amide (also known as teriflunomide, Formula I) for treating multiple sclerosis has been disclosed in U.S. Patent No. 6,794,410. Although aforesaid patent discloses that teriflunomide may possibly be combined with another compound known to be effective for treating multiple sclerosis to treat the disease, however, no specific combination is disclosed to show such efficacy and safety in treating multiple sclerosis.

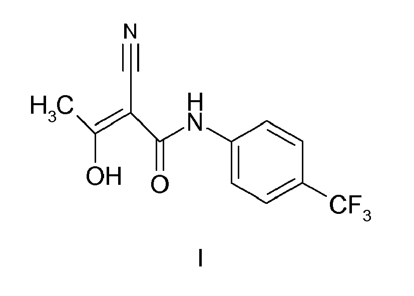

### Summary of the Invention

This invention is related to a method for treating multiple sclerosis, in a patient in need thereof, comprising administering to the patient about 7 mg or 14 mg of teriflunomide or a therapeutically equivalent amount of a pharmaceutically acceptable salt thereof, and a pharmaceutically effective amount of glatiramer acetate.

### Detailed Description of the Invention

As used above, and throughout the description of the invention, the following terms, unless otherwise indicated, shall be understood to have the following meanings:
"Patient" means a warm blooded animal, such as for example rat, mice, dogs, cats, guinea pigs, and primates such as humans.
"Pharmaceutically acceptable salts" means either an acid addition salt or a basic addition salt which is compatible with the treatment of patients for the intended use.
"Pharmaceutically acceptable acid addition salt" is any non-toxic organic or inorganic acid addition salt of the base compounds. Illustrative inorganic acids which form suitable salts include hydrochloric, hydrobromic, sulfuric and phosphoric acid and acid metal salts such as sodium monohydrogen orthophosphate and potassium hydrogen sulfate. Illustrative organic acids which form suitable salts include the mono-, di- and tri-carboxylic acids. Illustrative of such acids are, for example, acetic, glycolic, lactic, pyruvic, malonic, succinic, glutaric, fumaric, malic, tartaric, citric, ascorbic, maleic, hydroxymaleic, benzoic, hydroxybenzoic, phenylacetic, cinnamic, salicylic, 2-phenoxybenzoic, p-toluenesulfonic acid and sulfonic acids such as methanesulfonic acid and 2-hydroxyethanesulfonic acid. Either the mono- or di-acid salts can be formed, and such salts can exist in either a hydrated, solvated or substantially anhydrous form. In general, the acid addition salts of these compounds are more soluble in water and various hydrophilic organic solvents and which in comparison to their free base forms, generally demonstrate higher melting points.
"Pharmaceutically acceptable basic addition salts" means non-toxic organic or inorganic basic addition salts of the compounds. Examples are alkali metal or alkaline-earth metal hydroxides such as sodium, potassium, calcium, magnesium or barium hydroxides; ammonia, and aliphatic, alicyclic, or aromatic organic amines such as methylamine, trimethylamine and picoline. The selection criteria for the appropriate salt are known to one skilled in the art.
"Pharmaceutically effective amount" means an amount of a compound/composition according to the present invention effective in producing the desired therapeutic effect.
"Stable dose of glatiramer acetate" means administering, for example, about 20 mg glatiramer acetate per day, particularly by subcutaneous injection of about 20 mg once a day.
"Treat" or "treating" means to alleviate symptoms, eliminate the causation of the symptoms either on a temporary or permanent basis, or to prevent or slow the appearance of symptoms of the named disorder or condition.

One particular embodiment of the invention is a method for treating multiple sclerosis, in a patient in need thereof, comprising administering to the patient about 7 mg or 14 mg of teriflunomide once a day, and a stable dose of glatiramer acetate.

Another particular embodiment of the invention is related to a clinically proven safe and effective method for treating multiple sclerosis, in a patient in need thereof, comprising administering to the patient about 7 mg or 14 mg of teriflunomide once a day, and a stable dose of glatiramer acetate.

Another particular embodiment of the invention is related to a method for treating multiple sclerosis, in a patient in need thereof, comprising administering to the patient about 7 mg of teriflunomide once a day and about 20 mg of glatiramer acetate daily.

Another particular embodiment of the invention is related to a method for treating multiple sclerosis, in a patient in need thereof, comprising administering to the patient about 14 mg of teriflunomide once a day and about 20 mg of glatiramer acetate daily.

Another particular embodiment of the invention is related to a method for treating multiple sclerosis, wherein the teriflunomide or the pharmaceutically acceptable salt is administered orally.

Another particular embodiment of the invention is related to a method for treating multiple sclerosis, wherein the glatiramer acetate is administered subcutaneously.

Another particular embodiment of the invention is related to a method for reducing the number and volume of T1-Gd lesions in a patient afflicted with multiple sclerosis, comprising administering to the patient about 7 mg or 14 mg of teriflunomide or a therapeutically equivalent amount of a pharmaceutically acceptable salt thereof, and a pharmaceutically effective amount of glatiramer acetate.

Another particular embodiment of the invention is related to a method for reducing the number and volume of T1-Gd lesions in a patient afflicted with multiple sclerosis, comprising administering to the patient about 7 mg or 14 mg of teriflunomide once a day, and a stable dose of glatiramer acetate.

Another particular embodiment of the invention is related to a clinically proven safe and effective method for reducing the number and volume of T1-Gd lesions in a patient afflicted with multiple sclerosis, comprising administering to the patient about 7 mg or 14 mg of teriflunomide once a day, and a stable dose of glatiramer acetate.

Another particular embodiment of the invention is related to a method for reducing the number and volume of T1-Gd lesions in a patient afflicted with multiple sclerosis, comprising administering to the patient about 7 mg or 14 mg of teriflunomide or a therapeutically equivalent amount of a pharmaceutically acceptable salt thereof, and a pharmaceutically effective amount of glatiramer acetate, wherein the method reduces more T1-Gd lesions in the patient than when the patient is administered a stable dose of glatiramer acetate alone.

Another particular embodiment of the invention is related to a method for reducing the number and volume of T1-Gd lesions in a patient afflicted with multiple sclerosis, comprising administering to the patient about 7 mg or 14 mg of teriflunomide once a day, and a stable dose of glatiramer acetate, wherein the method reduces more T1-Gd lesions in the patient than when the patient is administered a stable dose of glatiramer acetate alone.

Another particular embodiment of the invention is related to a clinically proven safe and effective method for reducing the number and volume of T1-Gd lesions in a patient afflicted with multiple sclerosis, comprising administering to the patient about 7 mg or 14 mg of teriflunomide once a day, and a stable dose of glatiramer acetate, wherein the method reduces more T1-Gd lesions in the patient than when the patient is administered a stable dose of glatiramer acetate alone.

Another particular embodiment of the invention is related to a method for reducing the number and volume of T1-Gd lesions in a patient afflicted with multiple sclerosis, comprising administering to the patient about 7 mg of teriflunomide once a day and about 20 mg of glatiramer acetate daily.

Another particular embodiment of the invention is related to a method for reducing the number and volume of T1-Gd lesions in a patient afflicted with multiple sclerosis, comprising administering to the patient about 14 mg of teriflunomide once a day and about 20 mg of glatiramer acetate daily.

Another particular embodiment of the invention is related to a method for reducing the number and volume of T1-Gd lesions in a patient afflicted with multiple sclerosis, wherein the teriflunomide or the pharmaceutically acceptable salt is administered orally.

Another particular embodiment of the invention is related to reducing the number and volume of T1-Gd lesions in a patient afflicted with multiple sclerosis, wherein the glatiramer acetate is administered subcutaneously.

It is found that the addition of teriflunomide to stable-dosed glatiramer acetate improves disease control beyond glatiramer acetate alone, in terms of MRI activity, without added concerns regarding safety and tolerability.

### Example

A placebo-controlled, double-blinded, randomized study was conducted in relapsing-remitting multiple sclerosis patients who were concurrently on a stable dose of glatiramer acetate. The dose of glatiramer acetate was 20 mg subcutaneously daily.

Around 40 patients were treated in each treatment group (placebo: 41, 7 mg: 42, and 14 mg: 40). The demographic and disease baseline characteristics were generally comparable amount the 3 treatment groups. The mean age of the study population was 41.4 years. The majority of patients were female (78.9%) and had a relapsing remitting type of MS 94.3%), with a diagnosis of MS since around 8 years prior and approximately 41% of patients had no relapse in the previous year. The base line Expanded Disability Status Scale (EDSS) score was similar between treatment groups (around 2.5). A few more patients had Magnetic Resonance Image (MRI) disease activity (at least one T1-Gd lesion) at baseline in 7 mg group (28.6%) than in placebo (14.6%) and 14 mg (12.8%) groups.

### Method:

Safety was evaluated with reported Treatment Emergent Adverse Events (TEAE), physical examination and laboratory data (e.g. Liver Function Test, every 2-8 weeks), ECG and abdominal ultrasound (at baseline and at 24-weeks). Brain MRI activity, including T1-gadolinium (T1-Gd) lesions with central reading, as well as relapses and EDSS scores were recorded every 8-weeks.

### Results:

Safety: Tolerability and safety of each teriflunomide dose combined with glatiramer acetate were acceptable during the 24-week study (TEAE incidence rates: placebo: 78.0%, 7 mg: 83.3%, 14 mg: 87.5%). The number of patients with TEAE leading to treatment discontinuation was 0 in placebo; 3 (7.1%) in 7 mg; and 4 (10%) in 14 mg. Around 90% of patients completed the 24-week treatment period (placebo: 95.1%, 7 mg: 88.1 %, and 14 mg: 85.0%).

The proportion of patients with ALT (alanine aminotransferase) greater than 3ULN (three times above the Upper Limit of Normal range for the central laboratory) was low and similar across treatment groups (placebo: 1; 7 mg: 0; 14 mg: 1). The proportion of patients with reported TEAE of increased ALT was low and well balanced (two cases in each, including placebo). No case of concurrent increase in ALT > 3ULN and in total bilirubin > 2ULN was observed.

The proportion of patients with TEAE potentially related to immunosuppression was balanced for placebo (44%) and 7 mg group (43%) with slightly lower frequency in 14 mg group (38%). The proportion of patients with occurrence of decreased WBC (white blood cell) count and neutrophils (PCSA) was slightly higher in the two teriflunomide groups.

No patients died in this study.

Efficacy: The proportion of patient with at least one T1-Gd lesion on the baseline MRI scan was unbalanced among the groups, with a greater number of patients with T1-Gd activity in 7 mg group than in placebo and 14 mg groups (14.6% in placebo, 28.6% in 6 mg, and 12.8% in 14 mg), as was the number of lesion per patient (baseline-scan) (0.220 in placebo, 0.738 in 7 mg, and 0.333 in 14 mg).

Over 24 weeks, the mean number of lesions per scan decreased in both teriflunomide groups and increased in placebo (0.459 in placebo, 0.329 in 7 mg and 0.250 in 14 mg). This was seen also in the mean number of lesions per patient ("Patient Gd-enhancing T1-lesions per scan").

Similarly the mean number of lesions per scan and per patient, a numerical trend toward treatment effect was seen with the Poisson variable (mean number of lesion per scan), whether unadjusted or adjusted on planned covariates (regions and baseline number of lesions). The main analysis on number of T1-Gd lesions over 24 weeks using Poisson model with regions and baseline number of lesions as covariates indicated a relative risk reduction of 70.2% at 7 mg (p=0.010) and 53.6% at 14mg (p=0.1157).

This drug effect was reflected by the greater number of patients without Gd-enhancing lesions over 24-week at 14mg (73.2% in placebo and 81.6% in 14mg). The higher baseline activity in 7mg group may have contributed to the observed smaller number of patients without activity (61.0%). Analysis of total volume (ml) of Gd-enhancing T1-lesions per MRI scan using a permutation test led to similar results.

Conclusion: The addition of teriflunomide to stable-dosed glatiramer acetate improved disease control beyond glatiramer acetate alone, as evaluated by T1-Gd MRI activity, with acceptable safety and tolerability over 24 weeks of treatment.

## Claims

1. A method for treating multiple sclerosis, in a patient in need thereof, comprising administering to the patient about 7 mg or about 14 mg of teriflunomide, or a therapeutically equivalent amount of a pharmaceutically acceptable salt thereof, and a pharmaceutically effective amount of glatiramer acetate.

2. The method according to claim 1, wherein the patient is administered about 7 mg or about 14 mg of teriflunomide once a day, and a stable dose of glatiramer acetate.

3. A clinically proven safe and effective method for treating multiple sclerosis, in a patient in need thereof, comprising administering to the patient about 7 mg or 14 mg teriflunomide once a day, and a stable dose of glatiramer acetate.

4. A method for reducing the number and volume of T1-Gd lesions in a patient afflicted with multiple sclerosis comprising administering to the patient about 7 mg or 14 mg of teriflunomide or a therapeutically equivalent amount of a pharmaceutically acceptable salt thereof, and a pharmaceutically effective amount of glatiramer acetate.

5. The method according to claim 4, wherein the patient is administered about 7 mg or 14 mg of teriflunomide once a day, and a stable dose of glatiramer acetate.

6. A clinically proven safe and effective method for reducing the number and volume of T1-Gd lesions in a patient afflicted with multiple sclerosis comprising administering to the patient about 7 mg or 14 mg of teriflunomide once a day, and a stable dose of glatiramer acetate.

7. A method for reducing the number and volume of T1-Gd lesions in a patient afflicted with multiple sclerosis, comprising administering to the patient about 7 mg or 14 mg of teriflunomide or a therapeutically equivalent amount of a pharmaceutically acceptable salt thereof, and a pharmaceutically effective amount of glatiramer acetate, wherein the method reduces more T1-Gd lesions in the patient than when the patient is administered a stable dose of glatiramer acetate alone.

8. The method according to claim 7, wherein the patient is administered about 7 mg or 14 mg of teriflunomide once a day, and a stable dose of glatiramer acetate, and the method reduces more T1-Gd lesions in the patient than when the patient is administered a stable dose of glatiramer acetate alone.

9. A clinically proven safe and effective method for reducing the number and volume of T1-Gd lesions in a patient afflicted with multiple sclerosis, comprising administering to the patient about 7 mg or 14 mg of teriflunomide once a day, and a stable dose of glatiramer acetate, wherein the method reduces more T1-Gd lesions in the patient than when the patient is administered a stable dose of glatiramer acetate alone.

10. The method according to claims 1-9, wherein the patient is administered about 7 mg of teriflunomide once a day and about 20 mg of glatiramer acetate daily.

11. The method according to claims 1-9, comprising administering to the patient about 14 mg of teriflunomide once a day and about 20 mg of glatiramer acetate daily.

12. The method according to claims 1-9, wherein the teriflunomide or the pharmaceutically acceptable salt is administered orally.

13. The method according to claims 1-9, wherein the glatiramer acetate is administered subcutaneously.
